# EUROPÄISCHE PATENTANMELDUNG

(11) **EP 0 571 748 A2**
(43) Veröffentlichungstag der Anmeldung: **01.12.1993**
(21) Anmeldenummer: 93105800.2
(22) Anmeldetag: 08.04.1993
(51) Int. Cl.: C12N 11/04, C12N 11/08

(54) **Biokatalysator auf Polyurethan-Basis**

(30) Priorität: 29.05.1992 DE 4217891
(71) Anmelder: HERBERT SEUS GmbH & CO. SYSTEMTECHNIK KG, 26389 Wilhelmshaven (DE)
(72) Erfinder: Vorlop, Klaus Dieter, Prof. Dr., W-3300 Braunschweig (DE); Beyersdorf, Jörg-Henning, Dr., W-3300 Braunschweig (DE); Muscat, Andreas, Dipl.-Chem., W-2900 Oldenburg (DE)
(74) Vertreter: Lins, Edgar, Dipl.-Phys. Dr.jur.

(57) **Zusammenfassung**

Biokatalysatoren mit immobilisierten Zellen oder Enzymen, bei denen die Zellen oder Enzyme auf einer Matrix auf der Basis eines Polyurethans mit verkappten Isocyanat-Gruppen immobilisiert sind, sind durch Einbringen einer ersten Lösung eines Prepolymers in eine zweite Lösung eines Vernetzungsmittels in dreidimensionaler Form herstellbar.

## Beschreibung

Die Erfindung betrifft einen Biokatalysator mit immobilisierten Zellen oder Enzymen, bei dem die Zellen oder Enzyme in einer Matrix auf der Basis eines Polyurethans mit verkappten Isocyanat-Gruppen immobilisiert sind sowie ein Verfahren zur Herstellung eines derartigen Biokatalysators.

Biokatalysatoren mit immobilisierten Zellen oder Enzymen finden in den unterschiedlichsten Gebieten Anwendung. Die Anwendung bietet sich besonders in solchen Bereichen an, in denen die Zellen oder Enzyme nicht in dem durch Biotransformation erzeugten Produkt verbleiben können oder sollen, so z.B. bei der alkoholischen Gärung, bei der Herstellung von Arzneimitteln oder bei der Abwasserreinigung.

Matrizen aus Polyurethan-Hydrogelen weisen zwar sehr gute mechanische Eigenschaften auf, haben allerdings den großen Nachteil, daß die zu ihrer Herstellung eingesetzten Urethanprepolymere aufgrund der vorhandenen freien Isocyanatgruppen eine hohe Toxizität gegenüber Zellen und Enzymen aufweisen. Die hohe Toxizität führt in der Regel zu einer mehr oder minder starken Schädigung der Zellen bei der Immobilisierung und dadurch zu einem Aktivitätsverlust der mit diesen Urethanprepolymeren hergestellten Biokatalysatoren im Vergleich zu freien Zellen.

Zudem ist die Verarbeitung der Urethanprepolymere durch die kurze Verarbeitungszeit starken Einschränkungen unterworfen, bedingt durch die schnellen Vernetzungsreaktionen im Bereich von Sekunden.

Aus der JP 205 127 ist ein Verfahren zur Immobilisierung von Mikroorganismen bekannt, bei dem wasserlösliche Urethanprepolymer-Bisulfit-Addukte eingesetzt werden. Diese Urethanprepolymer-Bisulfit-Addukte werden nach diesem Verfahren in einer wässrigen Pufferlösung mit einem pH >9 verarbeitet. Wegen der kurzen zur Verfügung stehenden Verarbeitungszeit wird die Reaktionslösung zur Herstellung eines Biokatalysators auf eine Membran aufgesprüht.

Diese Membranen haben allerdings den großen Nachteil, daß sie wegen ihrer geringen mechanischen Stabilität häufig nicht einsetzbar sind. Es besteht daher weiterhin ein Bedarf nach Biokatalysatoren in dreidimensionaler, stabiler Form, z. B. als Kugeln oder Fäden.

Die der Erfindung zugrunde liegende Problemstellung ergibt sich daraus, daß ein Biokatalysator der eingangs erwähnten Art nur außerordentlich beschränkt einsetzbar ist.

Ausgehend von dieser Problemstellung ist der erfindungsgemäße Biokatalysator der eingangs erwähnten Art dadurch gekennzeichnet, daß die Zellen oder Enzyme in einer wässrigen Lösung des Urethan-Bisulfit-Adukt mit einem ersten Vernetzungspartner, der mit einem nicht in der Lösung befindlichen zweiten Vernetzungspartner reagieren kann, bei einem pH-Wert < 7 suspendiert werden, daß die so gebildete Suspension in eine den zweiten Vernetzungspartner enthaltende Flüssigkeit eingebracht wird und daß bei oder nach Ausbildung einer vernetzten Form der pH-Wert auf > 7 zur Vernetzung des Urethan-Bisulfit-Addukts (PCS) angehoben wird.

Der erfindungsgemäße Biokatalysator hat daher eine für Biokatalysatoren übliche Form und ist gegenüber bekannten Alginat-Katalysatoren mechanisch wesentlich stabiler. Der erfindungsgemäße Biokatalysator kann in einer biologisch nicht abbaubaren Konfiguration erstellt werden, wenn ein PCS auf der Basis eines Polyetherpolyols verwendet wird. Ist hingegen eine biologische Abbaubarkeit erwünscht, kann dies durch Ausbildung des PCS auf der Basis eines Polyesterpolyols erreicht werden.

Die aufgrund der geringen Verarbeitungszeit der polyurethan-Prepolymere bisher nicht mögliche Herstellung von sphärischen Biokatalysatoren gelingt mit speziellen, erfindungsgemäßen Verfahren.

Das erfindungsgemäße Verfahren beruht auf der Erkenntnis, daß die verkappten Polyurethan-Prepolymere, die für Zellen oder Enzyme nicht mehr toxisch sind, bei einem pH-Wert < 7 nicht oder nur langsam vernetzen, d. h. verarbeitbar bleiben. In dieser verarbeitbaren Form wird durch Einbringen der Suspension in die zweite Flüssigkeit sofort eine Formstabilisierung durch eine Vernetzungsreaktion zwischen den beiden Vernetzungspartnern erreicht. Diese Vernetzungsreaktion betrifft noch nicht die Vernetzung des Polyurethans. Erst wenn durch die Vernetzung stabile Formen, beispielsweise Kugeln oder Fäden sichergestellt sind, findet auch die Vernetzung des Polyurethans statt, indem der den Biokatalysator umgebende pH-Wert angehoben wird. Regelmäßig wird dies dadurch geschehen, daß die zweite Flüssigkeit auf einen pH-Wert > 7 eingestellt ist. In diesem Fall muß darauf geachtet werden, daß die Vernetzungsreaktion zwischen den beiden Vernetzungspartnern schneller abläuft als die Vernetzung des Polyurethans. Das Einleiten des Polyurethan-Prepolymers in eine zweite Flüssigkeit mit einem pH-Wert zwischen 7 und 10 ohne eine formgebende Maßnahme würde nicht zu brauchbaren Biokatalysatorformen führen.

Das Material für die beschriebenen Verfahren ist PCS.

In einer Ausführungsform des Verfahrens kann das ionische Vernetzungsmittel der Suspension zugegeben werden und die zweite Flüssigkeit die gelbildende Flüssigkeit aufweisen. In diesem Fall entsteht beim Eintropfen oder Eindüsen der Suspension in die zweite Flüssigkeit eine Vernetzung von innen nach außen, also von der Suspension in die zweite Flüssigkeit hinein, wodurch die zweite Flüssigkeit eine Hülle um die Suspension herum ausbildet. Beim Eintropfen der Suspension entstehen Hohlkugeln, die die Form der Suspension beim Aushärten des Polyurethans halten. Es entsteht somit ein vernetzter Polyurethankern mit einer Hülle aus beispielsweise Alginat. Diese Hülle kann anschließend entfernt werden.

Ein weiteres Verfahren besteht darin, als Vernetzungspartner zwei miteinander vernetzende Monomere zu verwenden, die beim Einbringen der Suspension in die zweite Flüssigkeit eine Grenzschicht-Polymerisation bewirken und somit ebenfalls eine Hülle aus dem gebildeten Polymer um die Suspension herum ausbilden und so die Form der Suspension während der Polyurethanaushärtung bestimmen. Auch diese Hülle kann nach der Aushärtung des Polyurethans entfernt werden.

Es ist weiterhin möglich, zur Formgebung, d.h. zur Ausbildung einer stabilisierenden Hülle, zwei Polyelektrolyte als Vernetzungspartner zu verwenden. Dabei befindet sich in der Suspension zweckmäßigerweise ein quartäres Ammoniumsalz wie z.B. Polydimethyldiallylammoniumchlorid. Die zweite Flüssigkeit enthält dann ein weiteres Polyelektrolyt, das beim Einleiten der Suspension sofort mit dem in der Suspension befindlichen Polyelektrolyten vernetzt, wodurch die Form des Katalysators stabilisiert wird. Im Fall des oben erwähnten Polydimethyldiallylammoniumchlorids kann z.B. Cellulosesulfat als Vernetzungsmittel verwendet werden. Die Vernetzung des Polyurethans erfolgt im Anschluß an die Ausbildung der Hülle ebenfalls durch Einwirkung des pH-Wertes, der vorzugsweise zwischen 7 und 10 eingestellt ist. Die durch Polyelektrolyt-Polyelektrolyt-Vernetzung gebildete Hülle ist so dünn, das sie bei diesem Verfahren nicht entfernt werden muß.

Ein bevorzugtes Einbringen der Suspension in die zweite Flüssigkeit erfolgt durch Eintropfen. Dabei entstehen für alle beschriebenen Verfahren Kugel- oder Tropfenformen für den Biokatalysator.

Alternativ dazu ist es möglich, die Suspension als kontinuierlichen Flüssigkeitsstrahl in die zweite Flüssigkeit einzuleiten, wodurch ein fadenförmiger Biokatalysator erzeugbar ist.

Ferner ist es möglich, mit der Suspension mit einer zentralen Düse einen Tropfen auszubilden, der bei seiner Entstehung mit der zweiten Flüssigkeit umgeben wird. Dies ist durch eine die erste zentrale Düse umgebende ringförmige Düse möglich, also mit der Anordnung einer bekannten Zweistoffdüse.

Weiterhin ist es mit der bekannten Zweistoffdüse möglich, einen als Hohlkugel oder Hohlfaser ausgebildeten Biokatalysator herzustellen. Dazu wird eine Zellsuspension, die ggfs. ein Mittel zur Erhöhung der Viskosität enthält, aus einer zentralen Düse ausgetragen, während gleichzeitig aus einer die zentrale Düse umgebenden ringförmigen Düse eine Lösung, die das PCS-Prepolymer und z.B. Ca-Ionen enthält, gleichzeitig ausgetragen wird, so daß der aus der ersten zentralen Düse austretende Flüssigkeitstropfen oder -strahl von der aus der zweiten ringförmigen Düse austretenden Flüssigkeit umschlossen wird. Die Vernetzung erfolgt dann auf die bereits beschriebene Art durch eine zweite Flüssigkeit.

Der besondere Vorteil dieses Verfahrens liegt in der Ausbildung einer Hohlkugel, deren Gelschicht zellfrei ist und in deren Inneren eine ungehinderte Vermischung des durch die Gelschicht hineindiffundierenden Substrats mit der Suspension möglich ist.

Das PCS-Prepolymer ist bei einem pH-Wert < 2 und bei einer Temperatur unter - 18° C unbeschränkt lagerfähig. Es kann für alle Verfahren vor der Verarbeitung auf einen für die Zellen oder Enzyme verträglichen pH-Wert von 4 bis 6 eingestellt werden.

Für die Verarbeitung der Urethanprepolymere und die Formgebung des Biokatalysators ist die Gelzeit und die Viskosität der die Zellen oder Enzyme enthaltenden Prepolymer-Suspension von besonderer Bedeutung. Wichtig ist außerdem die Wasserlöslichkeit der Prepolymere, damit auf den Einsatz von Cosolventien verzichtet werden kann.

Im Einklang mit bereits 1959 veröffentlichten Ergebnissen (Bailey Jr., F.E., Collard, R.W., J. Appl. Polym. Sci. 1 (1959) Nr.1, S. 59-62) wurde herausgefunden, daß mit steigendem Polyethylenoxid-Gehalt der aus Polyethylenoxid- und Polypropylenoxid-Einheiten zusammengesetzten Polymerketten der hier untersuchten Urethanprepolymere die Wasserlöslichkeit zunimmt, während bei zunehmendem Molekulargewicht und bei steigender Salzkonzentration in der Lösung die Wasserlöslichkeit erwartungsgemäß abnimmt. Dementsprechend werden in einer vorteilhaften Ausgestaltung des erfindungsgemäßen Verfahrens Urethanprepolymere eingesetzt, die einen Polyethylenoxid-Anteil von über 70 % aufweisen.

Durch Viskositätsmessung wurde ermittelt, in welchem Viskositätsbereich ein optimales Tropfverhalten zur Herstellung annähernd sphärischer Biokatalysatoren erzielt wird. Erfindungsgemäß werden dementsprechend in einer bevorzugten Ausgestaltung Prepolymer-Konzentrationen von vorzugsweise 10 - 20 % zu dem obengenannten Biokatalysator verarbeitet. Insbesondere werden in besonders bevorzugten Ausgestaltungen des Verfahrens die Konzentrationen an Prepolymeren auf 15 % bei dem pH von 4 (PCS 15-4), 12.5 % bei einem pH von 5 (PCS 12.5-5) und auf 10 % bei einem pH von 6 (PCS 10-6) eingestellt.

Durch Verwendung der Urethanprepolymer-Bisulfit-Addukte, andere Bezeichnung Polycarbamoylsulfonate (PCS), entsprechend dem vorliegenden Verfahren, ist es möglich, Biokatalysatoren herzustellen, deren Aktivität zumindest vergleichbar mit der von Biokatalysatoren auf der Basis von Biopolymeren, wie Ca-Alginat, Kalium-Karrageenan etc. ist. Dieser vorteilhafte Effekt beruht nicht nur auf der durch die Reaktion mit Bisulfit erfolgten Verkappung der reaktiven und toxischen Isocyanatgruppen, sondern auch, weil die "Verkappungsreaktion" in einem Wasser/Isopropanol-Gemisch erfolgt und das Isopropanol nach der Reaktion weitestgehend entfernt werden kann, wobei in der Praxis geringe Isopropanol-Rückstände in dem PCS-Prepolymer verbleiben, die praktisch keinen toxischen Einfluß auf die zu immobilisierenden Zellen oder Enzyme ausüben.

Die Erfindung soll im folgenden anhand von Beispielen im Zusammenhang mit den Zeichnungen näher erläutert werden. Es zeigen:
- Figur 1: ein Diagramm der Zellaktivität von S. cerevisiae;
- Figur 2: ein Diagramm zur Abhängigkeit der Gelzeit von dem pH-Wert;
- Figur 3: ein Diagramm zum optimalen Tropfverhalten am Beispiel von drei verschiedenen PCS-Lösungen;
- Figur 4: ein Schema zur Herstellung von PCS-Kugeln;
- Figur 5a: ein Diagramm zum Vergleich der Ethanolbildung durch S. cerevisiae in verschiedenen Polymermatrizes;
- Figur 5b: ein Diagramm der relativen Ethanolbildungsaktivität von S.cerevisiae in einer Ca-Alginat- und einer Polyurethan-Matrix;
- Figur 6: ein Diagramm zum Vergleich der Nitratabbauaktivität immobilisierter Zellen von P. denitrificans in Matrizes auf Basis von verschiedenen NCO-Prepolymeren;
- Figur 7a: ein Diagramm des Ammoniumabbaus mit freien und immobilisierten Nitrifikanten;
- Figur 7b: ein Diagramm zur relativen Ammoniumabbauaktivität von Biokatalysatoren auf der Basis von Polyurethan und Ca-Alginat.

Zur Herstellung der PCS-Prepolymere werden 20 g Polyetherpolyol mit einem Molekulargewicht von 2000 bis 4000 und einem Polyethylenoxid-Anteil > 70 % und 1.56 g TDI 80 (Toluylendiisocyanat, Isomerenverhältnis 2,4/2,6 ist 80/20) vermischt und in einem verschlossenen PE-Gefäß 25 Stunden auf 75°C gehalten.

Das auf diese Weise erhaltene Urethanprepolymer (PU-1) wird anschließend in Isopropanol gelöst. Zu einer Mischung von 40 g Prepolymer auf 60 g Isopropanol wird unter starkem Rühren eine Mischung aus 4.1 g Na-Bisulfit in 68 g destilliertem Wasser gegeben. Nach 15 Minuten wird die Reaktion durch Einstellen des pH-Wertes auf 1.5 mittels konzentrierter Salzsäure abgebrochen. Anschließend wird das Isopropanol bei 100 mbar und 40°C abgezogen.

Der Wassergehalt des so erhaltenen Produktes wird rechnerisch und der Sulfitgehalt titrimetrisch bestimmt. Der pH-Wert des Produktes wird nötigenfalls wieder auf 1.5 korrigiert, damit es während der Lagerung nicht zu einem Viskositätsanstieg des PCS-Prepolymers aufgrund von Vernetzungsreaktionen kommt.

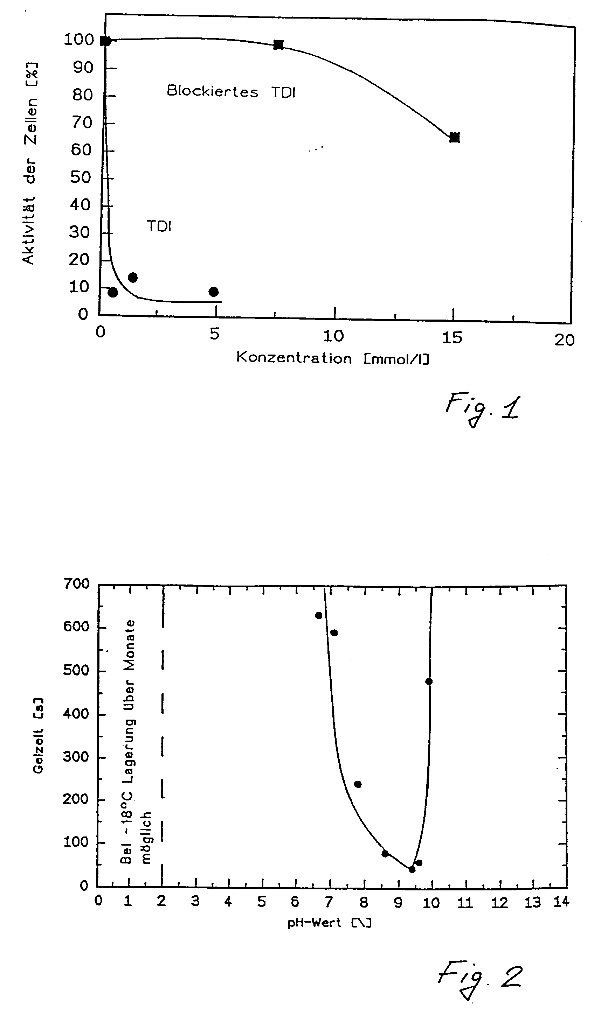

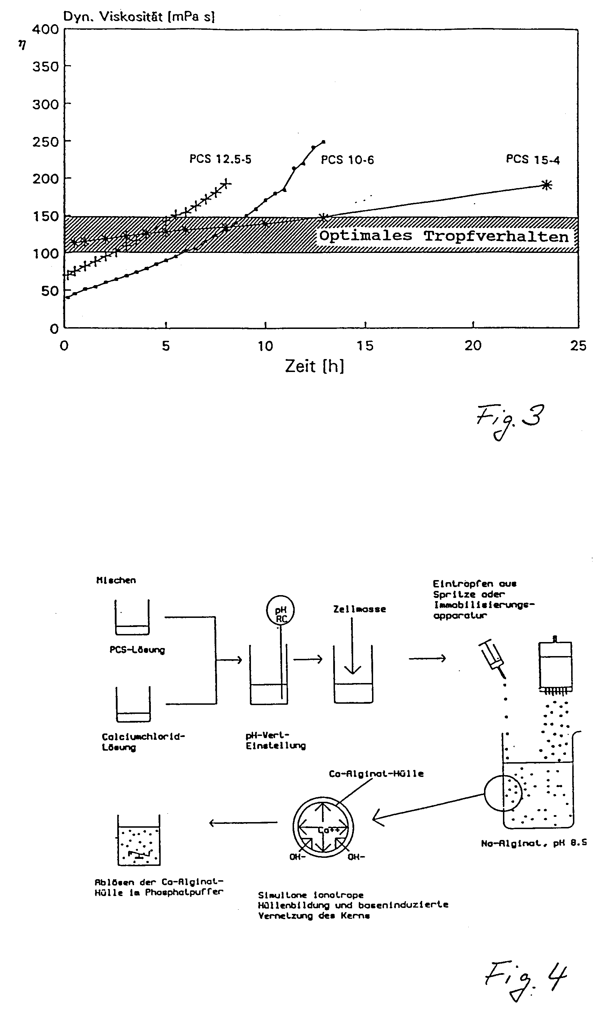

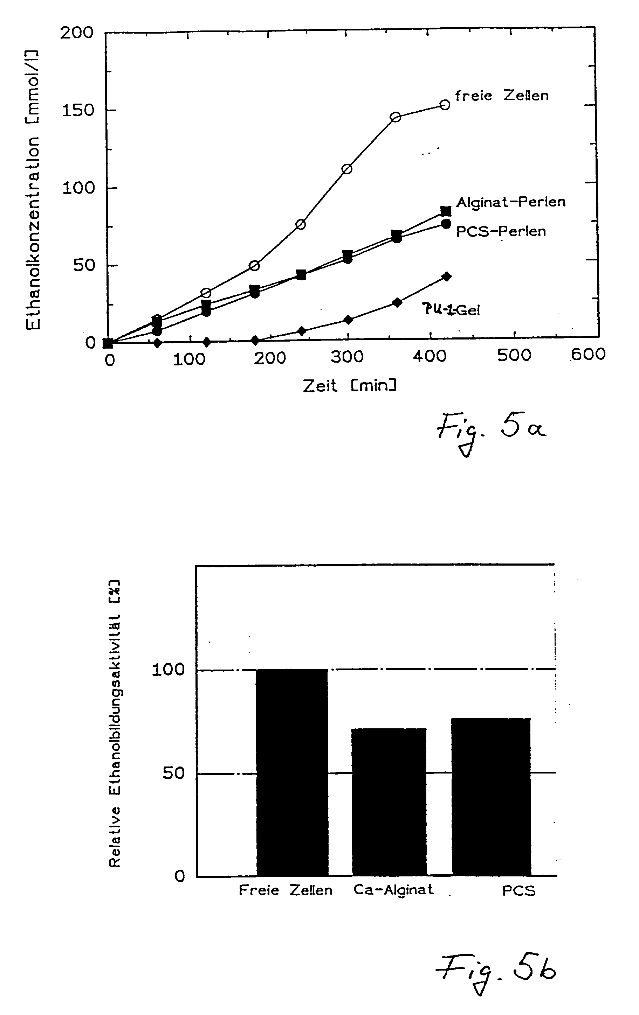

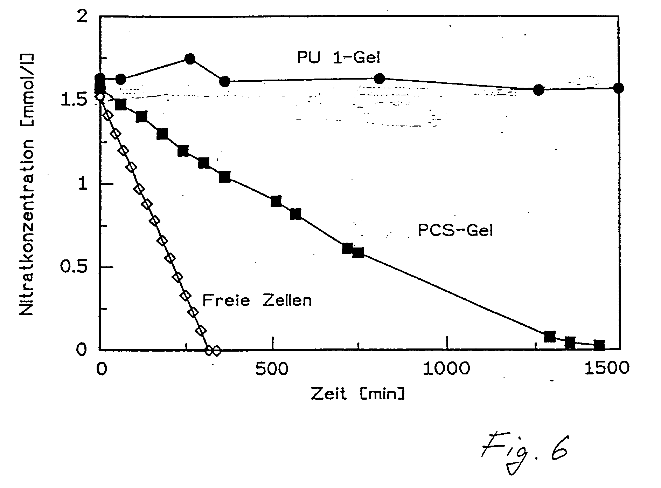

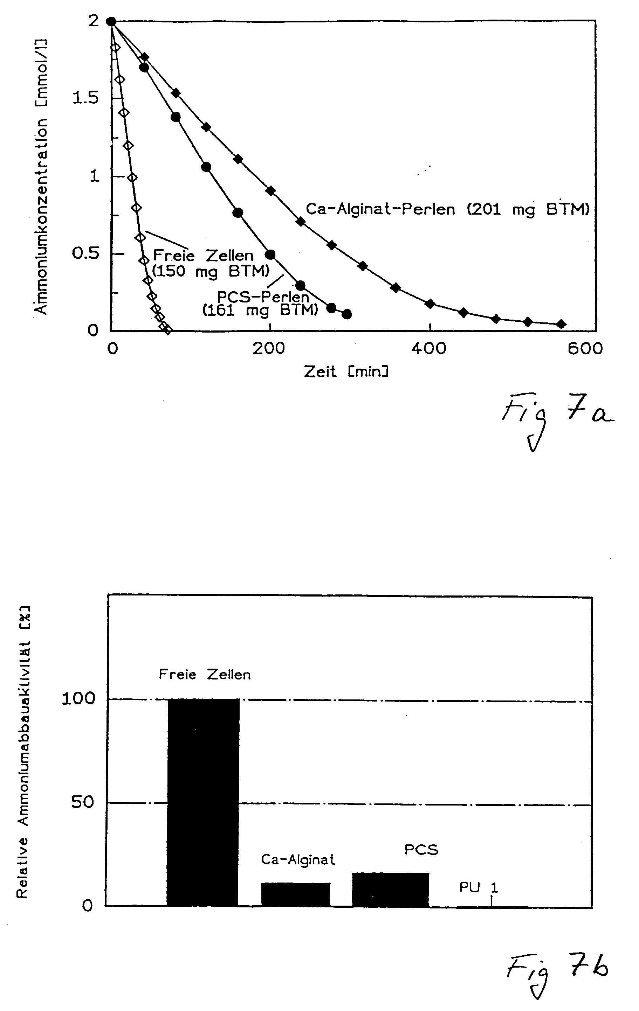

Um den Einfluß der Verkappung auf die Toxizität der Isocyanatgruppen zu untersuchen, wurde die Wirkung von Toluylendiisosyanat und dem TDI-Bisulfit-Addukt auf Saccharomyces cerevisiae überprüft. Figur 1 zeigt dabei sehr deutlich, daß schon bei sehr geringen Konzentrationen an freiem TDI nur noch eine Restaktivität der Mikroorganismen zu verzeichnen ist, während der Einfluß des TDI-Bisulfit-Adduktes auf S. cerevisiae über einen großen Konzentrationsbereich sehr gering ist.

Die für die Verarbeitung des PCS-Prepolymers besonders wichtigen Parameter, Gelzeit und Viskosität, sind eingehend untersucht worden. Die Ergebnisse sind in den Figuren 2 und 3 dargestellt. Figur 2 zeigt, daß das PCS-Prepolymer bei einem pH < 7 bzw. > 10 nur sehr langsam aushärtet und somit in einem verarbeitungsfähigen Zustand ist. In dem pH-Bereich von 7 - 10 sinkt die Vernetzungszeit rapide ab und findet ihr Minimum bei einem pH von etwas über 9, bei dem sie im Sekundenbereich liegt. Der optimale pH-Bereich für die Vernetzungsreaktion ist ungefähr 8.5 bis 9.5.

In Figur 3 ist das Tropfverhalten von Lösungen mit verschiedenen PCS-Konzentrationen bei bestimmten pH-Werten dargestellt. Als Maß hierfür dient die dynamische Viskosität η in Abhängigkeit von der Meßdauer. Sie wurde in einem Rotationsviskosimeter (nach DIN) bei Dᵣₑₚ = 704 S⁻¹ gemessen. Für die drei vermessenen PCS-Lösungen ergibt sich hieraus, daß eine ausreichend lange Verarbeitungszeit erreicht wird. Eine ausreichend lange Verarbeitungszeit ist Voraussetzung, um die Form des jeweils herzustellenden Biokatalysators hinreichend beeinflussen zu können.

Um aus Polycarbamoylsulfonaten sphärische Partikel zu erzeugen, wird eine Lösung hergestellt, die 10 % PCS-Prepolymer, das nach Beispiel 1 dargestellt wurde, und 2 % CaCl₂ in Wasser enthält. Der pH-Wert dieser Lösung wird auf 6 eingestellt und die Zellmasse zur Herstellung des Biokatalysators zugemischt. Anschließend wird die Mischung in eine leicht alkalische Na-Alginat-Lösung eingetropft. Um die Tropfen bilden sich sofort Hüllen aus Ca-Alginat, die die Form stabilisieren. Aufgrund der Vernetzungsreaktion im PCS-Kern, die durch die Hydroxylionen, die durch die Ca-Alginat-Hülle diffundieren, ausgelöst wird, sinkt der pH-Wert durch Freisetzung von schwefliger Säure leicht in den sauren Bereich ab. Durch Gegentitrieren mit NaOH wird der pH der Lösung im leicht alkalischen Bereich gehalten.

Nach 15 Minuten können die erhaltenen Perlen aus der Na-Alginat-Lösung genommen und in eine andere Lösung eingebracht werden, die einen leicht alkalischen pH aufweist. Zur Aushärtung des Kerns verbleiben die Perlen 1 bis 2 Stunden in dieser Lösung, wobei wenigstens in der ersten Zeit mit NaOH nachtitriert wird, um den pH-Wert im leicht Alkalischen zu halten.

Im Anschluß daran wird die Alginathülle mit Phosphatpuffer oder durch Rühren entfernt.

In Figur 4 ist die in diesem Beispiel angewendete Verfahrensweise zur Herstellung von PCS-Perlen durch ionotrope Gelbildung graphisch dargestellt. Zuerst wird die PCS-Lösung mit einer CaCl₂₋Lösung vermischt und der pH-Wert der Mischung eingestellt. Anschließend wird die Zellmasse hinzugefügt und in der Lösung suspendiert. Der entscheidende Schritt zur Herstellung von PCS-Perlen erfolgt, indem die Suspension mittels einer Spritze oder einer bekannten Immobilisierungsapparatur in eine Na-Alginat-Lösung mit einem pH von 8.5 eingetropft wird. Die dabei stattfindende simultane ionotrope Hüllenbildung und baseninduzierte Vernetzung des Kerns ist noch einmal hervorgehoben. Entsprechend der eingezeichneten Diffusionsrichtung der Ca- und Hydroxyl-Ionen findet die Vernetzung der Ca-Alginat-Hülle von innen nach außen statt, während der Kern von außen nach innen vernetzt. Der letzte Verfahrensschritt ist das Ablösen der Ca-Alginat-Hülle, das nach dem vollständigen Aushärten des Kerns erfolgt.

Bei der umgekehrten Variante des Verfahrens wird eine Zellsuspension, die 5 % PCS-Prepolymer und 2 % Na-Alginat enthält und auf einen pH-Wert von 4,5 eingestellt ist, in eine Pufferlösung mit einem pH von 8,5, die mit 2% CaCl₂ versetzt ist, getropft. Beim Eintropfen entstehen Biokatalysator-Perlen, die nach ein bis zwei Stunden ausgehärtet sind.

Eine weitere Möglichkeit zur Stabilisierung der Form des Katalysators erfolgt thermisch. Dabei wird eine 15%ige Lösung von PCS-Prepolymer in Wasser auf ungefähr pH 6 voreingestellt. Diese Lösung wird aus einer Spritze in eine auf 40°C erwärmte, 0.1 n Pufferlösung (Phosphatpuffer, pH 8.5) eingetropft. Beim Eintropfen färben sich die Polymertropfen sofort milchig weiß und behalten ihre Form bei. Nach einiger Zeit sind die Perlen durch den leicht basischen pH-Wert ausgehärtet.

In einem weiteren Verfahren wird die stabilisierende Hülle durch Polyelektrolyt-Polyelektrolyt-Vernetzung gebildet. Dabei ist die Suspension auf pH 6 eingestellt und enthält 10 % PCS-Prepolymer sowie 1 % Polydimethyldiallylammoniumchlorid. Diese Lösung wird in eine zweite Lösung getropft, die auf pH 8.5 eingestellt ist und 1.5 % Zellulosesulfat enthält. Beim Eintropfen in die zweite Lösung bildet sich unter Formerhaltung sofort eine dünne Hülle aus. Die Vernetzung des Kerns erfolgt dann durch Diffusion der Hydroxylionen durch diese Hülle von außen nach innen. Die gebildete Hülle ist so dünn, daß sie den Stofftransport im und aus dem Katalysator nicht behindert. Sie braucht deshalb nicht entfernt zu werden.

Um einen Vergleich von den erfindungsgemäßen Katalysatoren mit solchen, die aus dem Stand der Technik bekannt sind zu ermöglichen, sind nachfolgend die Ergebnisse von drei Vergleichsuntersuchungen dargestellt:

In den Figuren 5a und 5b ist die Aktivität verschiedener Biokatalysatoren mit immobilisierten Zellen von Saccharomyces cerevisiae im Vergleich zu freien Zellen dargestellt. Während die Aktivität von Biokatalysatoren auf der Basis von Alginat bzw. PCS nur geringfügig voneinander abweichen, zeigen Biokatalysatoren auf der Basis von nicht verkappten Polyurethanprepolymeren (PU-1) eine deutlich geringere Aktivität.

Figur 5b zeigt deutlich, daß Biokatalysatoren aus PCS-Perlen in der Aktivität der Ethanolbildung eine konkurrenzfähige Alternative zu Ca-Alginat-Biokatalysatorperlen darstellen.

In der Figur 6 ist ein Vergleich der Nitratabbauaktivität von Paracoccus denitrificans in Matrizes auf Basis von verschiedenen NCO-Prepolymeren und dem PCS-Prepolymer dargestellt. Es zeigt sich ganz deutlich, daß die Biokatalysatoren auf PCS-Basis über eine relativ große Aktivität verfügen, während die Biokatalysatoren auf der Basis anderer Urethanprepolymere praktisch inaktiv sind.

In den Figuren 7a und 7b ist die Ammoniumabbauaktivität von immobilisierten Nitrifikanten (hauptsächlich Nitrobacter winogradskyi und Nitrosomonas europeae) dargestellt. Es zeigt sich, daß in diesem Fall die Aktivität von Biokatalysatoren auf PCS-Basis von anderen Immobilisaten nicht erreicht wird.

Es muß jedoch hervorgehoben werden, daß in allen Beispielen die Aktivität der freien Zellen größer ist als die der immobilisierten Zellen. Es muß angenommen werden, daß eine gewisse Schädigung der Zellen durch den Immobilisierungsschritt für die Aktivitätseinbuße ausschlaggebend ist. Diese Aktivitätseinbuße tritt auch bei der gemeinhin als sehr schonend bezeichneten ionotropen Gelbildung mit Ca-Alginat auf. Biokatalysatoren auf PCS-Basis zeigen dabei in der Regel eine vergleichbare oder sogar größere Aktivität als Biokatalysatoren auf der Basis von Ca-Alginat. Einzig bei der Nitratabbauaktivität konnten Biokatalysatoren auf Basis von PCS die Aktivität von Biokatalysatoren auf Ca-Alginat nicht erreichen. Im Gegensatz dazu zeigen Biokatalysatoren auf der Basis von nicht verkappten Urethanprepolymeren in allen Fällen eine verschwindend geringe bzw. gar keine Aktivität.

## Patentansprüche

1. Verfahren zur Herstellung eines Biokatalysator mit Zellen oder Enzymen, die in einer Urethan-Bisulfit-Adukt-Matrix (Polycarbamoylsulfat-Matrix) bei einem pH-Wert > 7 immobilisiert werden, **dadurch gekennzeichnet**, **daß** die Zellen oder Enzyme in einer wässrigen Lösung des Urethan-Bisulfit-Adukt mit einem ersten Vernetzungspartner, der mit einem nicht in der Lösung befindlichen zweiten Vernetzungspartner reagieren kann, bei einem pH-Wert < 7 suspendiert werden, daß die so gebildete Suspension in eine den zweiten Vernetzungspartner enthaltende Flüssigkeit eingebracht wird und daß bei oder nach Ausbildung einer vernetzten Form der pH-Wert auf > 7 zur Vernetzung des Urethan-Bisulfit-Addukts (PCS) angehoben wird.

2. Verfahren nach Anspruch 1, dadurch gekennzeichnet, daß durch Verwendung eines ionischen Vernetzungsmittels als ersten Vernetzungspartner und einer ionotropen gelbildenden Flüssigkeit als zweiten Vernetzungspartner eine die Suspension umgebende Hülle erzeugt wird.

3. Verfahren nach Anspruch 1, dadurch gekennzeichnet, daß als erste und zweite Vernetzungspartner Monomere verwendet werden; die um die Suspension eine Hülle durch Grenzflächenpolymerisation bilden.

4. Verfahren nach Anspruch 2 oder 3, dadurch gekennzeichnet, daß nach der Vernetzung des Polyurethans die Hülle entfernt wird.

5. Verfahren nach einem der Ansprüche 1 bis 4, dadurch gekennzeichnet, daß mit der Suspension Tropfen gebildet werden, die in die zweite Flüssigkeit fallengelassen werden.

6. Verfahren nach einem der Ansprüche 1 bis 4, dadurch gekennzeichnet, daß mit der Suspension ein kontinuierlicher Flüssigkeitsstrahl erzeugt wird, der in die zweite Flüssigkeit eingeleitet wird.

7. Verfahren nach einem der Ansprüche 1 bis 6, dadurch gekennzeichnet, daß der pH-Wert der Suspension ≦ 6 eingestellt wird.

8. Verfahren nach Anspruch 7, dadurch gekennzeichnet, daß der pH-Wert der Suspension zwischen 4 und 6 eingestellt wird.

9. Verfahren nach einem der Ansprüche 1 bis 8, dadurch gekennzeichnet, daß der pH-Wert > 7 zur Vernetzung des Polyurethans > 8 gewählt wird.

10. Verfahren nach Anspruch 9, dadurch gekennzeichnet, daß der pH-Wert > 7 zur Vernetzung des Polyurethans bei etwa 9 eingestellt wird.

11. Verfahren nach einem der Ansprüche 1 bis 10, dadurch gekennzeichnet, daß das PCS auf der Basis eines Polyetherpolyols gebildet ist.

12. Verfahren nach einem der Ansprüche 1 bis 10, dadurch gekennzeichnet, daß das Polyurethan auf der Basis eines Polyesterpolyols gebildet ist.

13. Biokatalysator herstellbar mit einem Verfahren nach einem der Ansprüche 1 bis 12, bei dem Zellen oder Enzyme in einer reinen PCS-Matrix in einer dreidimensionalen festen Form immobilisiert sind.
